(19) **Europäisches Patentamt / European Patent Office / Office européen des brevets**

(11) **EP 3 548 521 B1**

(12) **FASCICULE DE BREVET EUROPEEN**

(45) Date de publication et mention
de la délivrance du brevet:
**26.03.2025 Bulletin 2025/13**

(21) Numéro de dépôt: **17822561.1**

(22) Date de dépôt: **01.12.2017**

(51) Classification Internationale des Brevets (IPC):
**C08F 2/22** $^{(2006.01)}$      **B01J 13/02** $^{(2006.01)}$
**B01J 13/14** $^{(2006.01)}$

(52) Classification Coopérative des Brevets (CPC):
**C08F 2/22; A01N 25/28; A61K 8/11; A61K 8/895;
A61Q 19/00; B01J 13/14; C09B 67/0078;
C09B 67/009;** A61K 9/5026; A61K 9/5031;
A61K 9/5089; A61K 2800/10

(86) Numéro de dépôt international:
**PCT/EP2017/081273**

(87) Numéro de publication internationale:
**WO 2018/100196 (07.06.2018 Gazette 2018/23)**

(54) **PROCÉDÉ DE PRÉPARATION DE MICROCAPSULES ET DE MICROPARTICULES**

VERFAHREN ZUR HERSTELLUNG VON MICROKAPSELN UND MICROPARTIKELN

METHOD TO PREPARE MICROCAPSULES AND MICROPARTICLES

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **01.12.2016 FR 1661794**

(43) Date de publication de la demande:
**09.10.2019 Bulletin 2019/41**

(73) Titulaire: **Calyxia
94380 Bonneuil-sur-Marne (FR)**

(72) Inventeurs:
• **WALTERS, Jamie
94380 Bonneuil sur Marne (FR)**

• **DEMOULIN, Damien
75014 Paris (FR)**

(74) Mandataire: **Lavoix
2, place d'Estienne d'Orves
75441 Paris Cedex 09 (FR)**

(56) Documents cités:
EP-A1- 1 502 646      EP-A1- 3 144 058
EP-A1- 3 144 059      US-A1- 2012 076 843
US-B2- 6 951 836

Remarques:
Le dossier contient des informations techniques
présentées postérieurement au dépôt de la
demande et ne figurant pas dans le présent
fascicule.

# Description

La présente invention concerne un procédé de préparation de microparticules et de microcapsules de taille contrôlée, en l'absence de photopolymérisation.

Dans de nombreuses industries, notamment l'industrie chimique, cosmétique, agrochimique, de la peinture ou des carburants et lubrifiants, il est important d'encapsuler et d'isoler un matériau actif du milieu environnant, afin de protéger l'actif contre l'hydrolyse, la dégradation thermique, l'oxydation ou encore d'autres procédés qui peuvent réduire la performance de l'actif. En outre, de nombreuses applications au sein de ces industries exigent que les particules ou les capsules produites aient une plage de taille étroite typiquement dans la gamme du micromètre (notamment entre 0,1 $\mu$m et 20 $\mu$m) par exemple pour avoir un meilleur contrôle sur leur performance globale.

Dans de nombreux produits fabriqués par l'industrie chimique comme les produits cosmétiques, les peintures, les revêtements ou les mastics, il est ainsi parfois ajouté des microparticules de polymère afin d'en modifier les propriétés rhéologiques ou la texture. Les performances rhéologiques atteintes sont extrêmement dépendantes de la taille moyenne des microparticules ainsi que de la plage de taille dans laquelle elles s'étendent (communément appelée la distribution de taille).

La problématique liée à l'isolement d'un actif du milieu environnant afin d'améliorer la performance des actifs est un domaine relativement nouveau pour un certain nombre d'industries. Dans la plupart des industries non biologiques, les pertes de performances associées à des facteurs tels que l'hydrolyse, la dégradation thermique, l'oxydation et la réactivité croisée sont résolues en augmentant la concentration de la matière active pour atteindre le niveau désiré de performance, ce qui augmente le coût et génère également d'autres problèmes associés au produit formé à partir de tels procédés.

Ces dernières années, un grand nombre de procédés de fabrication de particules ou de capsules ont été développés et rapportés dans la littérature, y compris le séchage par atomisation, l'évaporation de solvant, la polymérisation interfaciale et l'extrusion centrifuge parmi beaucoup d'autres. Cependant, pour les procédés de fabrication de particules et de capsules à l'échelle industrielle, les techniques d'émulsion dominent. De tels procédés ont recours à une étape formant une émulsion d'une huile hydrophobe ou d'une phase cireuse, dispersée dans un milieu aqueux ou alternativement une phase aqueuse, dispersée dans une huile hydrophobe ou un milieu cireux. Les deux phases sont émulsifiées en utilisant soit un homogénéisateur, soit un récipient agité équipé de chicanes et stabilisé en utilisant des tensioactifs, des lipides ou des émulsifiants polymériques. Alternativement, une réaction à l'interface entre les deux phases peut être utilisée pour la formation d'une enveloppe de polymère.

Toutefois, ces systèmes produisent des émulsions et des capsules qui sont polydisperses ou de taille trop élevée (au-dessus de 20 $\mu$m).

En outre, ces systèmes requièrent l'utilisation d'eau pour former l'une des phases. Ils requièrent également l'utilisation de tensioactifs ou émulsifiants similaires pour stabiliser l'émulsion, qui présentent l'inconvénient de pouvoir réagir avec l'encapsulant ou fournir des contaminants dans les différentes phases.

La présente invention a pour but de fournir des particules et des capsules contenant un actif par la mise en œuvre d'un procédé en masse afin de satisfaire les volumes pour répondre aux demandes des industries non biologiques.

La présente invention a également pour but de fournir un procédé par émulsification permettant d'obtenir des particules et des capsules de taille contrôlée, notamment de taille inférieure à 20 $\mu$m, voire 5 $\mu$m.

La présente invention a également pour but de fournir un procédé d'encapsulation d'actifs pouvant être mis en œuvre en l'absence d'eau et/ou de tensioactifs et d'émulsifiants.

La présente invention a également pour but de fournir un procédé de fabrication de microparticules et de microcapsules au moyen d'émulsions constituées de matériaux qui rendent les gouttes qui les composent d'une part monodisperses et d'autre part extrêmement stables.

Ainsi, la présente invention concerne un procédé de préparation de microparticules et de microcapsules solides comprenant les étapes suivantes :

a) l'addition sous agitation d'une composition C'2 dans une composition C3, les compositions C'2 et C3 n'étant pas miscibles l'une dans l'autre,

la composition C'2 étant soit une composition C2 monophasique réticulable soit une émulsion (E1) comprenant des gouttes d'une composition C1, comprenant au moins un actif, dispersées dans une composition polymérique C2 réticulable, les compositions C1 et C2 n'étant pas miscibles l'une dans l'autre, la viscosité de la composition C3 étant supérieure à 10 000 mPa.s à 25°C à une vitesse de cisaillement de 10 s$^{-1}$ et étant inférieure à 10 000 mPa.s à 25°C à une vitesse de cisaillement comprise entre 100 s$^{-1}$ et 100 000 s$^{-1}$, ce par quoi on obtient une émulsion (E2) comprenant des gouttes de composition C'2 dispersées dans la composition C3 ;

b) l'application d'un cisaillement à l'émulsion (E2), ladite vitesse de cisaillement appliquée étant comprise entre 100 s$^{-1}$ et 100 000 s$^{-1}$, ce par quoi on obtient une émulsion (E3) comprenant des gouttes de composition C'2 de taille contrôlée dispersées dans la composition C3 ; et

c) la polymérisation de la composition C'2, ce par quoi on obtient des microparticules ou des microcapsules solides dispersées dans la composition C3, et pendant l'étape c), l'émulsion (E3) est laissée au repos pendant une période pouvant aller jusqu'à 100 heures.

Selon la nature de la composition C'2, le procédé de l'invention permet d'obtenir des microparticules ou des microcapsules. Lorsque la composition C'2 comprend au moins un actif, on parlera alors de microcapsules.

En fonction de la nature de la composition C'2, l'émulsion (E2) est soit une émulsion simple comprenant des gouttes de composition C2 dispersées dans la composition C3, soit une émulsion double comprenant des gouttes dispersées dans la composition C3 (correspondant à l'émulsion (E'2) mentionnée plus loin).

En fonction de la nature de la composition C'2, l'émulsion (E3) est soit une émulsion simple comprenant des gouttes de taille contrôlée de composition C2 dispersées dans la composition C3, soit une émulsion double comprenant des gouttes de taille contrôlée dispersées dans la composition C3 (correspondant à l'émulsion (E'2) mentionnée plus loin).

Selon un mode de réalisation, le procédé de l'invention comprend les étapes suivantes :

a") l'addition sous agitation d'une composition C2 réticulable dans une composition C3, les compositions C2 et C3 n'étant pas miscibles l'une dans l'autre,

la viscosité de la composition C3 étant supérieure à 10 000 mPa.s à 25°C à une vitesse de cisaillement de 10 s$^{-1}$ et étant inférieure à 10 000 mPa.s à 25°C à une vitesse de cisaillement comprise entre 100 s$^{-1}$ et 100 000 s$^{-1}$, ce par quoi on obtient une émulsion (E2) comprenant des gouttes de composition C2 dispersées dans la composition C3 ;

b) l'application d'un cisaillement à l'émulsion (E2), ladite vitesse de cisaillement appliquée étant comprise entre 100 s$^{-1}$ et 100 000 s$^{-1}$, ce par quoi on obtient une émulsion (E3) comprenant des gouttes de composition C2 de taille contrôlée dispersées dans la composition C3 ; et
c) la polymérisation de la composition C2, ce par quoi on obtient des microparticules solides dispersées dans la composition C3, et pendant l'étape c), l'émulsion (E3) est laissée au repos pendant une période pouvant aller jusqu'à 100 heures.

Selon ce mode de réalisation, le procédé de l'invention permet ainsi la production à l'échelle industrielle de populations de gouttes d'émulsion de taille contrôlée et notamment inférieure à 20 $\mu$m. Le contrôle de la taille des gouttes obtenues par le procédé de l'invention est dû en particulier au contrôle des propriétés rhéologiques de la phase continue C3.

Selon ce mode de réalisation, le procédé de l'invention permet la production de microparticules de taille contrôlée par la mise en œuvre d'une étape de polymérisation des gouttes d'émulsion. Cette étape de polymérisation permet notamment de solidifier les gouttes et élimine ainsi toute coalescence.

Selon un autre mode de réalisation, la composition C'2 susmentionnée est une émulsion (E1) obtenue par l'addition sous agitation d'une composition C1, comprenant au moins un actif, dans une composition polymérique C2 réticulable, les compositions C1 et C2 n'étant pas miscibles l'une dans l'autre.

Selon ce mode de réalisation, le procédé de l'invention comprend les étapes suivantes :

a1) l'addition sous agitation d'une composition C1, comprenant au moins un actif, dans une composition C2 réticulable, les compositions C1 et C2 n'étant pas miscibles l'une dans l'autre, ce par quoi on obtient une émulsion (E1) comprenant des gouttes de composition C1 dispersées dans la composition C2 ;
a2) l'addition sous agitation de l'émulsion (E1) dans une composition C3, les compositions C2 et C3 n'étant pas miscibles l'une dans l'autre,

la viscosité de la composition C3 étant supérieure à 10 000 mPa.s à 25°C à une vitesse de cisaillement de 10 s$^{-1}$ et étant inférieure à 10 000 mPa.s à 25°C à une vitesse de cisaillement comprise entre 100 s$^{-1}$ et 100 000 s$^{-1}$, ce par quoi on obtient une émulsion double (E'2) comprenant des gouttes dispersées dans la composition C3 ;

b) l'application d'un cisaillement à l'émulsion (E'2), ladite vitesse de cisaillement appliquée étant comprise entre 100 s$^{-1}$ et 100 000 s$^{-1}$, ce par quoi on obtient une émulsion double (E'3) comprenant des gouttes de taille contrôlée dispersées dans la composition C3 ; et
c) la polymérisation de la composition C2, ce par quoi on obtient des microcapsules solides dispersées dans la composition C3, et pendant l'étape c), l'émulsion (E'3) est laissée au repos pendant une période pouvant aller jusqu'à 100 heures.

Selon ce mode de réalisation, le procédé de l'invention permet ainsi la production à l'échelle industrielle de populations de gouttes d'émulsion double de taille contrôlée et notamment inférieure à 20 $\mu$m. Le contrôle de la taille des gouttes obtenues par le procédé de l'invention est dû en particulier au contrôle des propriétés rhéologiques de la phase continue C3.

Selon ce mode de réalisation, le procédé de l'invention permet la production de capsules de taille contrôlée par la mise en œuvre d'une étape de polymérisation de la phase intermédiaire de la double émulsion. Cette étape de polymérisation permet notamment de solidifier la couche intermédiaire des capsules et élimine ainsi toute coalescence.

De préférence, les microparticules et les microcapsules obtenues selon le procédé de l'invention ont un diamètre moyen (tel que mesuré par microscopie optique ou par MET ou par technique de diffusion de la lumière) compris entre 0,1 $\mu$m et 20 $\mu$m, et de préférence entre 1 $\mu$m et 20 $\mu$m.

### Etape a)

Pendant l'étape a), une composition C'2 est ajoutée à une composition C3, cette étape étant effectuée sous agitation, ce qui signifie que la composition C3 est agitée, typiquement de façon mécanique, tandis que la composition C'2 est ajoutée, et ce afin d'émulsifier le mélange des compositions C'2 et C3.

L'addition de la composition C'2 dans la composition C3 est typiquement effectuée goutte à goutte.

Selon un mode de réalisation, la composition C'2 peut être une émulsion (E1) telle que définie ci-dessus. Cette émulsion (E1) est préparée selon l'étape a1) telle que décrite ci-dessous.

Pendant l'étape a1), une composition C1 est ajoutée à une composition C2 réticulable, cette étape étant effectuée sous agitation, ce qui signifie que la composition C2 est agitée, typiquement de façon mécanique, tandis que la composition C1 est ajoutée, et ce afin d'émulsifier le mélange des compositions C1 et C2.

L'addition de la composition C1 dans la composition C2 est typiquement effectuée goutte à goutte.

Pendant l'étape a1), la composition C1 est à une température comprise entre 0°C et 100°C, de préférence entre 10°C et 80°C, et préférentiellement entre 15°C et 60°C. Pendant l'étape a1), la composition C2 est à une température comprise entre 0°C et 100°C, de préférence entre 10°C et 80°C, et préférentiellement entre 15°C et 60°C.

Dans les conditions d'addition de l'étape a1), les compositions C1 et C2 ne sont pas miscibles l'une dans l'autre, ce qui signifie que la quantité (en poids) de la composition C1 capable d'être solubilisée dans la composition C2 est inférieure ou égale à 5%, de préférence inférieure à 1%, et préférentiellement inférieure à 0,5%, par rapport au poids total de composition C2, et que la quantité (en poids) de la composition C2 capable d'être solubilisée dans la composition C1 est inférieure ou égale à 5%, de préférence inférieure à 1%, et préférentiellement inférieure à 0,5%, par rapport au poids total de composition C1.

Ainsi, lorsque la composition C1 entre en contact avec la composition C2 sous agitation, celle-ci est dispersée sous la forme de gouttes, dites gouttes simples.

L'immiscibilité entre les compositions C1 et C2 permet également d'éviter la migration de l'actif de la composition C1 vers la composition C2.

La composition C2 est agitée de manière à former une émulsion comprenant des gouttes de composition C1 dispersées dans la composition C2. Cette émulsion est aussi appelée « émulsion simple » ou émulsion C1-dans-C2.

Pour mettre en œuvre l'étape a1), on peut utiliser tout type d'agitateur usuellement utilisé pour former des émulsions, comme par exemple un agitateur mécanique à pâles, un émulseur statique, un homogénéisateur à ultrasons, un homogénéisateur à membrane, un homogénéisateur à haute pression, un moulin colloïdal, un disperseur à haut pouvoir de cisaillement ou un homogénéisateur à haute vitesse.

### Composition C1

La composition C1 comprend au moins un actif A. Cette composition C1 sert de véhicule à l'actif A dans le procédé de l'invention, au sein des gouttes formées lors du procédé de l'invention et des capsules solides obtenues.

Selon une première variante du procédé de l'invention, la composition C1 est monophasique, c'est-à-dire qu'il s'agit de l'actif A pur ou bien d'une solution comprenant l'actif A sous forme solubilisée.

Selon un mode de réalisation, l'actif est solubilisé dans la composition C1.

Selon cette variante, la composition C1 consiste typiquement en une solution de l'actif A dans une solution aqueuse, ou un solvant organique, ou un mélange de solvants organiques, l'actif A étant présent selon une teneur massique comprise de 1% à 99%, par rapport à la masse totale de la composition C1. L'actif A peut être présent selon une teneur massique comprise de 5% à 95%, de 10% à 90%, de 20% à 80%, de 30% à 70%, ou de 40% à 60%, par rapport à la masse totale de la composition C1.

Selon un mode de réalisation, la composition C1 consiste en l'actif A.

Selon un autre mode de réalisation de l'invention, la composition C1 est une composition biphasique, ce qui signifie que l'actif est dispersé, soit sous forme liquide soit sous forme solide, dans la composition C1 et n'est pas totalement solubilisé dans ladite composition C1.

Selon un mode de réalisation, l'actif est dispersé sous la forme de particules solides dans la composition C1.

Selon ce mode de réalisation, la composition C1 peut consister en une dispersion de particules solides de l'actif dans un solvant organique ou dans un mélange de solvants organiques.

Selon ce mode de réalisation, la composition C1 peut consister en une dispersion de particules solides de l'actif dans une phase aqueuse, qui comprend de l'eau et éventuellement des solvants organiques hydrophiles.

L'actif utilisé est par exemple :

- un réticulant, un durcisseur, un catalyseur organique ou métallique (tel qu'un complexe organométallique ou inorganométallique de platine, de palladium, de titane, de molybdène, de cuivre, de zinc) utilisé pour polymériser des formulations de polymère, d'élastomère, de caoutchouc, de peinture, d'adhésif, de joint, de mortier, de vernis ou de revêtement ;
- un colorant ou un pigment destiné aux formulations d'élastomères, de peinture, de revêtement, d'adhésif, de joint, de mortier, ou de papier ;
- un parfum (au sens de la liste de molécule établie par l'International Fragrance Association (IFRA) et disponible sur le site internet www.ifraorg.org) destiné aux produits de détergence comme les lessives, aux produits de soin de la maison, aux produits cosmétiques et de soin de la personne, aux textiles, aux peintures, aux revêtements ;
- un arôme, une vitamine, un acide aminé, une protéine, un lipide, un probiotique, un antioxydant, un correcteur de pH, un préservateur pour les composés alimentaires et l'alimentation animale ;
- un adoucissant, un conditionnant pour les produits de détergence, les lessives, les cosmétiques et les produits de soin de la personne. A ce titre, les actifs utilisables sont par exemple énumérés dans les brevets US 6 335 315 et US 5 877 145 ;
- un agent anti altération de couleur (tel qu'un dérivé d'ammonium), un agent antimousse (tel qu'un éthoxylate d'alcool, un sulfonate d'alkylbenzène, un éthoxylate de polyéthylène, un alkyléthoxysulfate ou alkylsulfate) destiné aux produits de détergence et aux lessives et aux produits de soin de la maison ;
- un agent azurant, aussi appelé activateur de couleur (tel qu'un dérivé de stilbène, un dérivé de coumarine, un dérivé de pyrazoline, un dérivé de benzoxazole ou un dérivé de naphtalimide) destiné aux produits de détergence, aux lessives, aux cosmétiques et aux produits de soin de la personne ;
- un composé biologiquement actif tel qu'une enzyme, une vitamine, une protéine, un extrait végétal, un agent émollient, un agent désinfectant, un agent antibactérien, un agent anti-UV, un médicament destiné aux produits cosmétiques et de soin de la personne, aux textiles. Parmi ces composés biologiquement actifs on peut citer : les vitamines A, B, C, D et E, l'acide para aminobenzoïque, les acides alpha hydroxylés (comme l'acide glycolique, l'acide lactique, l'acide malique, l'acide tartrique ou l'acide citrique), le camphre, les céramides, les polyphénols (comme les flavonoïdes, l'acide phénolique, l'acide ellagique, le tocophérol, l'ubiquinol), l'hydroquinone, l'acide hyaluronique, l'isopropyl isostéarate, l'isopropyl palmitate, l'oxybenzone, le panthenol, la proline, le rétinol, le rétinyl palmitate, l'acide salicylique, l'acide sorbique, le sorbitol, le triclosan, la tyrosine ;
- un agent désinfectant, un agent antibactérien, un agent anti-UV, destiné aux peintures et revêtements ;
- un fertilisant, un herbicide, un insecticide, un pesticide, un fongicide, un repoussant ou un désinfectant destiné aux produits agrochimiques ;
- un agent ignifuge, aussi appelé retardateur de flamme, (tel qu'un polyol bromé comme le tetrabromobisphenol A, un composé organophosphoré halogéné ou non halogéné, un composé chloré, un trihydrate d'aluminium, un oxyde d'antimoine, un borate de zinc, un phosphore rouge, un mélamine, ou un dihydroxyde de magnesium) destiné aux matériaux plastiques, aux revêtement, aux peintures et aux textiles ;
- un cristal photonique ou un photochromophore destiné aux peintures, aux revêtements et aux matériaux polymères formant les écrans incurvés et souples ;
- un produit connu par l'homme de l'art sous le nom de matériaux à changement de phase (PCM pour Phase Change Materials) capables d'absorber ou restituer de la chaleur lorsqu'ils subissent un changement de phase, destinés au stockage d'énergie. Des exemples de PCM et de leurs applications sont décrits dans "A review on phase change energy storage: materials and applications", Farid et al., Energy Conversion and Management, 2004, 45(9-10), 1597-1615. Comme exemples de PCM, on peut citer les sels fondus de phosphate d'aluminium, le carbonate d'ammonium, le chlorure d'ammonium, le carbonate de césium, le sulfate de césium, le citrate de calcium, le chlorure de calcium, l'hydroxyde de calcium, l'oxyde de calcium, le phosphate de calcium, le saccharate de calcium, le sulfate de calcium, le phosphate de cérium, le phosphate de fer, le carbonate de lithium, le sulfate de lithium, le chlorure de magnésium, le sulfate de magnésium, le chlorure de manganèse, le nitrate de manganèse, le sulfate de manganèse, l'acétate de potassium, le carbonate de potassium, le chlorure de potassium, le phosphate de potassium, le carbonate de rubidium, le sulfate de rubidium, le tétraborate de disodium, l'acétate de sodium, le bicarbonate de sodium, le bisulfate de sodium, le citrate de sodium, le chlorure de sodium, l'hydroxyde de sodium, le nitrate de sodium, le percarbonate de sodium, le persulfate de sodium, le phosphate de sodium, le propionate de sodium, le sélénite de sodium, le silicate de sodium, le sulfate de sodium, le tellurate de sodium, le thiosulfate de sodium, l'hydrophosphate de strontium, l'acétate de zinc, le chlorure de zinc, le thiosulfate de sodium, les cires hydrocarbonées paraffiniques, les polyéthylène glycols.

Selon un mode de réalisation, la composition C'2 peut être une composition C2 telle que définie ci-dessus.

Selon ce mode de réalisation, l'étape a) correspond à l'étape a") décrite ci-dessus.

Pendant l'étape a"), une composition C2 réticulable est ajoutée à une composition C3, cette étape étant effectuée sous agitation, ce qui signifie que la composition C3 est agitée, typiquement de façon mécanique, tandis que la composition C2 est ajoutée, et ce afin d'émulsifier le mélange des compositions C2 et C3.

L'addition de la composition C2 dans la composition C3 est typiquement effectuée goutte à goutte.

Pendant l'étape a"), la composition C2 est à une température comprise entre 0°C et 100°C, de préférence entre 10°C et 80°C, et préférentiellement entre 15°C et 60°C. Pendant l'étape a"), la composition C3 est à une température comprise entre 0°C et 100°C, de préférence entre 10°C et 80°C, et préférentiellement entre 15°C et 60°C.

Dans les conditions d'addition de l'étape a"), les compositions C2 et C3 ne sont pas miscibles l'une dans l'autre, ce qui signifie que la quantité (en poids) de la composition C2 capable d'être solubilisée dans la composition C3 est inférieure ou égale à 5%, de préférence inférieure à 1%, et préférentiellement inférieure à 0,5%, par rapport au poids total de composition C3, et que la quantité (en poids) de la composition C3 capable d'être solubilisée dans la composition C2 est inférieure ou égale à 5%, de préférence inférieure à 1%, et préférentiellement inférieure à 0,5%, par rapport au poids total de composition C2.

Ainsi, lorsque la composition C2 entre en contact avec la composition C3 sous agitation, celle-ci est dispersée sous la forme de gouttes, dites gouttes simples.

L'immiscibilité entre les compositions C2 et C3 permet également d'éviter la migration de l'actif de la composition C2 vers la composition C3.

La composition C3 est agitée de manière à former une émulsion comprenant des gouttes de composition C2 dispersées dans la composition C3. Cette émulsion est aussi appelée « émulsion simple » ou émulsion C2-dans-C3.

Pour mettre en œuvre l'étape a"), on peut utiliser tout type d'agitateur usuellement utilisé pour former des émulsions, comme par exemple un homogénéisateur à ultrasons, un homogénéisateur à membrane, un homogénéisateur à haute pression, un moulin colloïdal, un disperseur à haut pouvoir de cisaillement ou un homogénéisateur à haute vitesse.

*Composition C2*

La composition C2 est une composition réticulable ce qui signifie qu'il s'agit d'une composition capable de polymériser (réticuler) pour donner un matériau solide, pour former l'enveloppe polymérisée des microcapsules solides de l'invention.

Selon l'invention, la composition C2 n'est pas une composition photoréticulable.

Selon un mode de réalisation, la composition C2 est un liquide dont la viscosité à 25°C est comprise entre 500 mPa.s et 100 000 mPa.s à une vitesse de cisaillement de 10 s$^{-1}$.

La viscosité est mesurée au moyen d'un rhéomètre Haake Rheostress™ 600 équipé d'un cône de diamètre 60 mm et d'angle 2 degrés, et d'une cellule de régulation en température réglée à 25°C.

De préférence, la viscosité de la composition C2 à 25°C est comprise entre 1 000 mPa.s et 50000 mPa.s à une vitesse de cisaillement de 10 s$^{-1}$, préférentiellement entre 2 000 mPa.s et 25 000 mPa.s, et par exemple entre 3 000 mPa.s et 15 000 mPa.s.

De préférence, la viscosité de la composition C2 est supérieure à la viscosité de la composition C1.

Selon ce mode de réalisation, indépendamment de la viscosité de l'actif ou de ses propriétés chimiques, la cinétique de déstabilisation des gouttes de l'émulsion (E1) est significativement lente, ce qui permet à l'enveloppe des microcapsules d'être polymérisée pendant l'étape d) avant que l'émulsion ne se déstabilise. La polymérisation, une fois achevée, fournit alors une stabilisation thermodynamique.

Ainsi, la viscosité relativement élevée de la composition C2 assure la stabilité de l'émulsion (E1) obtenue à l'issue de l'étape a).

De préférence, la tension interfaciale entre les compositions C1 et C2 est faible. Typiquement, ces tensions interfaciales varient entre 0 mN/m et 50 mN/m, de préférence entre 0 mN/m et 20 mN/m.

La faible tension interfaciale entre les compositions C1 et C2 permet également de façon avantageuse d'assurer la stabilité de l'émulsion (E1) obtenue à l'issue de l'étape a1).

Selon un mode de réalisation, le ratio entre le volume de composition C1 et le volume de composition C2 varie entre 1:10 et 10:1. De préférence, ce ratio est compris entre 1:3 et 5:1, préférentiellement entre 1:3 et 3:1.

Ce ratio peut être adapté afin de contrôler l'épaisseur de l'enveloppe des microcapsules polymérisées.

Selon un mode de réalisation, la composition C2 comprend au moins un monomère ou polymère, au moins un agent réticulant, et éventuellement au moins un catalyseur.

Selon un mode de réalisation avantageux, la composition C2 ne comprend pas de photoinitateur.

Selon un mode de réalisation, la composition C2 comprend de 50% à 99% en poids de monomère ou de polymère, ou un mélange de monomères ou polymères, par rapport au poids total de la composition C2.

Selon un mode de réalisation, la composition C2 comprend de 1% à 20% en poids d'agent réticulant ou d'un mélange d'agents réticulants, par rapport au poids total de la composition C2.

Selon un mode de réalisation, la composition C2 comprend de 0,001% à 5% en poids de catalyseur ou d'un mélange de catalyseurs, par rapport au poids total de la composition C2.

Selon un mode de réalisation, la composition C2 comprend de 0,001% à 70% en poids d'agent réticulant par rapport au poids total de composition C2.

Selon l'invention, le terme « monomère » ou « polymère » désigne toute unité de base adaptée pour la formation d'un matériau solide par polymérisation, soit seul soit en combinaison avec d'autres monomères ou oligomères.

Ces monomères peuvent être choisis parmi les monomères comprenant au moins une fonction réactive choisie dans le groupe constitué des fonctions acrylate, méthacrylate, vinyl éther, N-vinyl éther, mercaptoester, thiolène, siloxane, époxy, oxétane, uréthane, isocyanate et peroxyde.

En particulier, les monomères peuvent être choisis parmi les monomères portant au moins une des fonctions réactives susmentionnées et portant en outre au moins une fonction choisie dans le groupe constitué des fonctions alkylamines primaires, secondaires et tertiaires, des fonctions amines quaternaires, des fonctions sulfate, sulfonate, phophate, phosphonate, carboxylate, hydroxyle, halogène, et leurs mélanges.

Les polymères utilisés dans la composition C2 peuvent être choisis parmi les polyéthers, polyesters, polyuréthanes, polyurées, polyéthylène glycols, polypropylène glycols, polyamides, polyacétals, polyimides, polyoléfines, polysulfures et les polydiméthylsiloxanes, lesdits polymères portant en outre au moins une fonction réactive choisie dans le groupe constitué des fonctions acrylate, méthacrylate, vinyl éther, N-vinyl éther, mercaptoester, thiolène, siloxane, époxy, oxétane, uréthane, isocyanate et peroxyde.

Parmi les exemples de tels monomères ou oligomères, on peut citer, mais de façon non limitative, les polymères suivants : poly(2-(1-naphthyloxy)-éthyl acrylate), poly(2-(2-naphthyloxy)-éthyl acrylate), poly(2-(2-naphthyloxy)-éthyl méthacrylate), polysorbitol diméthacrylate, polyacrylamide, poly((2-(1-naphthyloxy) éthanol), poly(2-(2-naphthyloxy) éthanol), poly(1-chloro-2,3-époxypropane), poly(n-butyl isocyanate), poly(N-vinyl carbazole), poly(N-vinyl pyrrolidone), poly(p-benzamide), poly(p-chlorostyrène), poly(p-méthyl styrène), poly(p-phénylène oxide), poly(p-phénylène sulfure), poly(N-(méthacryloxyéthyl)succinimide), polybenzimidazol, polybutadiène, polybutylène téréphthalate, polychloral, polychloro trifluoro éthylène, polyéther imide, polyéther cétone, polyéther sulfone, polyhydridosilsesquioxane, poly(m-phénylène isophthalamide), poly(méthyl 2-acrylamido-2-méthoxyacéate), poly(2-acrylamido-2-méthylpropanesulfonique acide), poly-mono-butyl maléate, polybutylméthacrylate, poly(N-tert-butylméthacrylamide), poly(N-n-butylméthacrylamide), polycyclohexylméthacrylamide, poly(m-xylènebisacrylamide 2,3-diméthyl-1,3-butadiène,N,N-diméthylméthacrylamide), poly(n-butyl méthacrylate), poly(cyclohexyl méthacrylate), polyisobutyl méthacrylate, poly(4-cyclohexylstyrène), polycyclol acrylate, polycyclol méthacrylate, polydiéthyl éthoxyméthylènemalonate, poly(2,2,2-trifluoroéthyl méthacrylate), poly(1,1,1-triméthylolpropane triméthacrylate), polyméthacrylate, poly(N,N-diméthylaniline, dihydrazide), poly(dihydrazine isophthalique), polyacide isophthalique, polydiméthyl benzilketal, épichlorohydrine, poly(éthyl-3,3-diéthoxyacrylate), poly(éthyl-3,3-diméthylacrylate), poly(éthyl vinylcétone), poly(vinyl éthylcétone), poly(penten-3-one), polyformaldéhyde poly(diallyl acétal), polyfumaronitrile, polyglycéryl propoxy triacrylate, polyglycéryl triméthacrylate, polyglycidoxypropyltriméthoxysilane, polyglycidyl acrylate, poly(n-heptyl acrylate), poly(n-heptyl ester d'acide acrylique), poly(n-heptyl méthacrylate), poly(3-hydroxypropionitrile), poly(2-hydroxypropyl acrylate), poly(2-hydroxypropyl méthacrylate), poly(N-(méthacryloxyéthyl)phthalimide), poly(1,9-nonanediol diacrylate), poly(1,9-nonanediol diméthacrylate), poly(N-(n-propyl) acrylamide), poly(acide orthophthalique), poly(acide iso-phthalique), poly(acide 1,4-benzenedicarboxylique), poly(acide 1,3-benzenedicarboxylique), poly(acide phthalique), poly(mono-2-acryloxyéthyl ester), polyacide téréphthalique, polyanhydride phthalique, polyéthylène glycol diacrylate, polyéthylène glycol méthacrylate, polyéthylène glycol diméthacrylate, poly(isopropyl acrylate), polysorbitol pentaacrylate, polyvinyl bromoacétate, polychloroprène, poly(di-n-hexyl silylène), poly(di-n-propyl siloxane), polydiméthyl silylène, polydiphényl siloxane, polyvinyl propionate, polyvinyl triacétoxysilane, polyvinyl tris-tert-butoxysilane, polyvinyl butyral, polyalcool vinylique, polyacétate de vinyle, polyéthylène co-vinyl acétate, poly(bisphénol-A polysulfone), poly(1,3-dioxepane), poly(1,3-dioxolane), poly(1,4-phénylène vinylène), poly(2,6-diméthyl-1A-phénylène oxyde), poly(acide 4-hydroxybenzoique), poly(4-méthyl pentène-1), poly(4-vinyl pyridine), polyméthylacrylonitrile, polyméthylphénylsiloxane, polyméthylsilméthylène, polyméthylsilsesquioxane, poly(phénylsilsesquioxane), poly(pyromellitimide-1.4-diphényl éther), polytétrahydrofurane, polythiophène, poly(triméthylène oxyde), polyacrylonitrile, polyéther sulfone, polyéthylène-co-vinyl acétate, poly(perfluoréthylène propylène), poly(perfluoralkoxyl alcane), ou poly(styrène-acrylonitrile).

Par « agent réticulant », on entend un composé porteur d'au moins deux fonctions réactives susceptibles de réticuler un monomère ou un polymère, ou un mélange de monomères ou de polymères, lors de sa polymérisation.

L'agent réticulant peut être choisi parmi des molécules portant au moins deux fonctions choisies dans le groupe constitué des fonctions acrylate, méthacrylate, vinyl éther, N-vinyl éther, mercaptoester, thiolène, siloxane, époxy, oxétane, uréthane, isocyanate et peroxyde.

A titre d'agent réticulant, on peut notamment citer:

- les diacrylates, comme le 1,6-hexanediol diacrylate, le 1,6-hexanediol diméthacrylate, le polyéthylène glycol diméthacrylate, le 1,9-nonanediol diméthacrylate, le 1,4-butanediol diméthacrylate, le 2,2-bis(4-méthacryloxyphényl)propane, le 1,3-butanediol diméthacrylate, le 1,10-décanediol diméthacrylate, le bis(2-méthacryloxyéthyl) N,N'-1,9-nonylène biscarbamate, le 1,4-butanediol diacrylate, l'éthylène glycol diacrylate, le 1,5-pentanediol diméthacrylate, le 1,4-Phénylène diacrylate, l'allyl méthacrylate, le N,N'-méthylènebisacrylamide, le 2,2-bis[4-(2-hydroxy-3-méthacryloxypropoxy)phényl]propane, le tétraéthylène glycol diacrylate, l'éthylène glycol diméthacrylate, le diéthylène glycol diacrylate, le triéthylène glycol diacrylate, , le triéthylène glycol diméthacrylate, le polyéthylène glycol diglycidyl éther, le N,N-diallylacrylamide, le 2,2-bis[4-(2-acryloxyéthoxy)phényl]propane, le glycidyl méthacrylate ;

- les acrylates multifonctionnels comme le dipentaérythritol pentaacrylate, le 1,1,1- triméthylolpropane triacrylate, le 1,1,1-triméthylolpropane triméthacrylate, l'éthylènediamine tétraméthacrylate, le pentaérythritol triacrylate, le pentaérythritol tétraacrylate ;
- les acrylates possédant également une autres fonction réactive, comme le propargyl méthacrylate, le 2-Cyanoéthyl acrylate, le tricyclodécane diméthanol diacrylate, l'hydroxypropyl méthacrylate, le N-acryloxysuccinimide, le N-(2-Hydroxypropyl)méthacrylamide, le N-(3-aminopropyl)méthacrylamide hydrochloride, le N-(t-BOC-aminopropyl)methacrylamide, le 2-aminoéthyl méthacrylate hydrochloride, le monoacryloxyéthyl phosphate, le o-nitrobenzyl méthacrylate, l'anhydride acrylique, le 2-(tert-butylamino) ethyl méthacrylate, le N,N-diallylacrylamide, le glycidyl méthacrylate, le 2-hydroxyéthyl acrylate, le 4-(2-acryloxyaéhoxy)-2-hydroxybenzophenone, le N-(Phthalimidométhyl)acrylamide, le cinnamyl méthacrylate.

Le catalyseur peut être choisi parmi les catalyseurs organiques, métalliques, organométalliques ou inorganométalliques.

A titre de catalyseur, on peut notamment citer les complexes organométalliques ou inorganométalliques de platine, de palladium, de titane, de molybdène, de cuivre, de zinc.

Selon un mode de réalisation, la composition C2 peut en outre comprendre un monomère ou un polymère additionnel capable d'améliorer les propriétés de l'enveloppe des microcapsules et/ou de donner de nouvelles propriétés à l'enveloppe des microcapsules.

Parmi ces monomères ou polymères additionnels, on peut citer les monomères ou polymères portant un groupe sensible au pH, à la température, aux UV ou aux IR.

Ces monomères ou polymères additionnels peuvent induire la rupture des microcapsules solides et par la suite la libération de leur contenu, après une stimulation via le pH, la température, les UV ou les IR.

Ces monomères ou polymères additionnels peuvent être choisis parmi les monomères ou polymères portant au moins une fonction réactive choisie dans le groupe constitué des fonctions acrylate, méthacrylate, vinyl éther, N-vinyl éther, mercaptoester, thiolène, siloxane, époxy, oxétane, uréthane, isocyanate et peroxyde, et portant également l'un des groupes suivants :

- un groupe hydrophobe tel qu'un groupe fluoré, par exemple le trifluoroéthyl méthacrylate, le trifluoroéthyl acrylate, le tétrafluoropropyl méthacrylate, le pentafluoropropyl acrylate, le hexafluorobutyl acrylate, ou le fluorophényl isocyanate ;
- un groupe sensible au pH comme les amines primaires, secondaires ou tertiaires, les acides carboxyliques, les groupes phosphate, sulfate, nitrate, ou carbonate ;
- un groupe sensible aux UV ou clivable par UV (ou groupe photochromique) comme les groupes azobenzène, spiropyrane, 2-diazo-1,2-naphthoquinone, o-nitrobenzylé, thiol, ou 6-nitro-veratroyloxycarbonyle, par exemple poly(éthylène oxyde)-bloc-poly(2-nitrobenzylméthacrylate), et d'autres copolymères à blocs, comme décrit notamment dans Liu et al., Polymer Chemistry 2013, 4, 3431-3443 ;
- un groupe sensible aux IR ou clivable par IR comme le o-nitrobenzyle ou le 2-diazo-1,2-naphthoquinone, par exemple les polymères décrits dans Liu et al., Polymer Chemistry 2013, 4, 3431-3443 ; et
- un groupe sensible à la température comme le poly(N-isopropylacrylamide).

### *Etape a2)*

Pendant l'étape a2), l'émulsion (E1) obtenue à l'étape a') est ajoutée à une composition C3, cette étape étant effectuée sous agitation, ce qui signifie que la composition C3 est agitée, typiquement de façon mécanique, tandis que l'émulsion (E1) est ajoutée, et ce afin d'émulsifier le mélange des compositions C1, C2 et C3.

L'addition de l'émulsion (E1) dans la composition C3 est typiquement effectuée goutte à goutte.

Pendant l'étape a2), l'émulsion (E1) est à une température comprise entre 15°C et 30°C. Pendant l'étape a2), la composition C3 est à une température comprise entre 15°C et 30°C.

Dans les conditions d'addition de l'étape a2), les compositions C2 et C3 ne sont pas miscibles l'une dans l'autre, ce qui signifie que la quantité (en poids) de la composition C2 capable d'être solubilisée dans la composition C3 est inférieure ou égale à 5%, de préférence inférieure à 1%, et préférentiellement inférieure à 0,5%, par rapport au poids total de composition C3, et que la quantité (en poids) de la composition C3 capable d'être solubilisée dans la composition C2 est inférieure ou égale à 5%, de préférence inférieure à 1%, et préférentiellement inférieure à 0,5%, par rapport au poids total de composition C2.

Ainsi, lorsque l'émulsion (E1) entre en contact avec la composition C3 sous agitation, celle-ci est dispersée sous la forme de gouttes, dites gouttes doubles, la dispersion de ces gouttes d'émulsion (E1) dans la phase continue C3 étant appelée émulsion (E2').

Typiquement, une goutte double formée pendant l'étape a2) correspond à une goutte simple de composition C1 telle que décrite ci-dessus, entourée par une enveloppe de composition C2 qui encapsule totalement ladite goutte simple.

La goutte double formée pendant l'étape a2) peut également comprendre au moins deux gouttes simples de composition C1, lesdites gouttes simples étant entourées par une enveloppe de composition C2 qui encapsule totalement lesdites gouttes simples.

Ainsi, lesdites gouttes doubles comprennent un cœur constitué d'une ou plusieurs gouttes simples de composition C1, et une couche de composition C2 entourant ledit cœur.

L'émulsion (E'2) résultante est généralement une émulsion double polydisperse (émulsion C1-dans-C2-dans-C3 ou émulsion C1/C2/C3), ce qui signifie que les gouttes doubles n'ont pas une nette distribution de taille dans l'émulsion (E2').

L'immiscibilité entre les compositions C2 et C3 permet d'éviter le mélange entre la couche de composition C2 et la composition C3 et assure ainsi la stabilité de l'émulsion (E'2).

L'immiscibilité entre les compositions C2 et C3 permet également d'éviter à de l'actif de la composition C1 de migrer du noyau des gouttes vers la composition C3.

Pour mettre en œuvre l'étape a2), on peut utiliser tout type d'agitateur usuellement utilisé pour former des émulsions, comme par exemple un homogénéisateur à ultrasons, un homogénéisateur à membrane, un homogénéisateur à haute pression, un moulin colloïdal, un disperseur à haut pouvoir de cisaillement ou un homogénéisateur à haute vitesse.

### Composition C3

Selon l'invention, la viscosité de la composition C3 à 25°C est supérieure à 10 000 mPa.s à une vitesse de cisaillement de 10 s$^{-1}$ et inférieure à 10 000 mPa.s à la vitesse de cisaillement à laquelle est réalisée c'), c'est-à-dire à une vitesse de cisaillement comprise entre 100 s$^{-1}$ et 100 000 s$^{-1}$.

La composition C3 peut également être désignée comme une composition gélifiée.

De préférence, la viscosité de la composition C3 à 25°C est comprise entre 15 000 mPa.s et 100 000 mPa.s à une vitesse de cisaillement de 10 s$^{-1}$ et inférieure à 5 000 mPa.s à la vitesse de cisaillement à laquelle est réalisée c'), c'est-à-dire à une vitesse de cisaillement comprise entre 100 s$^{-1}$ et 100 000 s$^{-1}$.

De préférence, la tension interfaciale entre les compositions C2 et C3 est faible. La faible tension interfaciale entre les compositions C2 et C3 permet également de façon avantageuse d'assurer la stabilité de l'émulsion (E2) ou (E'2).

Selon un mode de réalisation, le ratio entre le volume d'émulsion (E1) et le volume de composition C3 varie entre 1:10 et 10:1. De préférence, ce ratio est compris entre 1:9 et 3:1, préférentiellement entre 1:9 et 1:1.

Ce ratio peut être adapté afin de contrôler la quantité totale de matériel actif encapsulé parmi la population résultante de microcapsules polymérisées.

Selon un mode de réalisation, la composition C3 comprend au moins un polymère branché, de préférence de poids moléculaire supérieur à 5 000 g.mol$^{-1}$ préférentiellement entre 10 000 g.mol$^{-1}$ et 500 000 g.mol$^{-1}$, par exemple entre 50 000 g.mol$^{-1}$ et 300 000 g.mol$^{-1}$.

Par « polymère branché » (ou polymère ramifié), on entend un polymère présentant au moins un point de ramification entre ses deux groupes terminaux, un point de ramification (aussi appelé point de branchement) étant un point d'une chaîne sur lequel est fixée une chaîne latérale aussi appelée branche ou chaîne pendante.

Parmi les polymères branchés, on peut par exemple citer les polymères greffés, en peigne ou encore les polymères en étoile ou les dendrimères.

Selon un mode de réalisation, la composition C3 comprend au moins un polymère de poids moléculaire supérieur à 5 000 g.mol$^{-1}$.

A titre de polymère utilisable dans la composition C3, on peut citer les composés suivants, utilisés seuls ou bien mélangés entre eux :

- les dérivés de cellulose, tels que les éthers de cellulose : le méthyl cellulose, l'éthyl cellulose, l'hydroxyéthyl cellulose, le méthylhydroxyéthyl cellulose, l'éthylhydroxyéthyl cellulose, le carboxyméthyl cellulose, l'hydroxypropyl cellulose ou le méthylhydroxypropyl cellulose ;

- les polyacrylates (encore appelés carbomères), tels que l'acide polyacrylique (PAA), l'acide polyméthacrylique (PMAA), le poly(hydroxyéthyl méthacrylate) (pHEMA), le poly(N-2-hydroxypropyl méthacrylate) (pHPMA) ;

- les polyacrylamides tels que le poly(N-isopropylacrylamide) (PNIPAM) ;

- le polyvinylpyrrolidone (PVP) et ses dérivés ;

- l'alcool polyvinylique (PVA) et ses dérivés ;

- le poly(éthylène glycol), le poly(propylène glycol) et leurs dérivés, tels que le poly(éthylène glycol) acrylate/méthacrylate, le poly(éthylène glycol) diacrylate/diméthacrylate, le polypropylène carbonate ;

- les polysaccharides tels que les carraghénanes, les gommes de caroube ou gommes tara, le dextran, les gommes xanthanes, le chitosane, l'agarose, les acides hyaluroniques, la gomme gellane, la gomme de guar, la gomme arabique, la gomme adragante, la gomme diutane, la gomme d'avoine, la gomme karaya, la gomme ghatti, la gomme curdlan, la pectine, la gomme konjac ;

- les dérivés protéinés tels que la gélatine, le collagène, la fibrine, la polylysine, l'albumine, la caséine ;
- les dérivés de silicone tels que le polydimethylsiloxane (aussi appelé diméthicone), les alkyl silicones, les aryl silicones, les alkyl aryl silicones, les polyéthylène glycol diméthicones, les polypropylène glycol diméthicone ;
- les cires, telles que les cires diester (diesters d'alcanediol, diesters d'hydroxylacides), les cires triester (triacylglycérols, triesters d'alcane-1,2-diol, de $\omega$-hydroxy acide et d'acide gras, esters d'acide hydroxymalonique, d'acide gras et d'alcool, triesters d'hydroxylacides, d'acide gras et d'alcool gras, triesters d'acide gras, d'hydroxylacide et de diol) et les cires polyesters (polyesters d'acides gras). Les esters d'acides gras utilisables à titre de cires dans le cadre de l'invention sont par exemple le palmitate de cétyle, l'octanoate de cétyle, le laurate de cétyle, le lactate de cétyle, l'isononanoate de cétyle, le stéarate de cétyle, le stéarate de stéaryle, le stéarate de myristyle, le myristate de cétyle, le stéarate d'isocétyle, le trimyristate de glycéryle, le tripalmitate de glycéryle, le monostéarate de glycéryle ou le palmitate de glycéryle et de cétyle ;
- les acides gras utilisables comme cires tels que l'acide cérotique, l'acide palmitique, l'acide stéarique, l'acide dihydroxystéarique, l'acide béhénique, l'acide lignocérique, l'acide arachidique, l'acide myristique, l'acide laurique, l'acide tridécyclique, l'acide pentadécyclique, l'acide margarique, l'acide nonadécyclique, l'acide hénéicosylique, l'acide tricosylique, l'acide pentacosylique, l'acide heptacosylique, l'acide montanique ou l'acide nonacosylique ;
- les sels d'acide gras notamment les sels d'aluminium d'acide gras tels que l'aluminium stéarate, l'hydroxyl aluminium bis(2-éthylhexanoate) ;
- l'huile de jojoba isomérisée ;
- l'huile de tournesol hydrogénée ;
- l'huile de coprah hydrogénée ;
- l'huile de lanoline hydrogénée ;
- l'huile de ricin et ses dérivés, notamment l'huile de ricin hydrogénée modifiée ou les composés obtenus par estérification d'huile de ricin avec des alcools gras ;
- les polyuréthanes et leurs dérivés ;
- les polymères styréniques tels que le styrène butadiène ; et
- les polyoléfines telles que le polyisobutène.

Selon un mode de réalisation, la composition C3 comprend des particules solides telles que des argiles, des silices et des silicates.

A titre de particules solides utilisables dans la composition C3, on peut citer les argiles et silicates appartenant notamment à la catégorie des phyllosilicates (encore appelées silices en feuillets). A titre d'exemple de silicate utilisable dans le cadre de l'invention, on peut citer la Bentonite, l'Hectorite, l'Attapulgite, la Sepiolite, la Montmorillonite, la Saponite, la Sauconite, la Nontronite, la Kaolinite, le Talc, la Sepiolite, la Craie. Les silices synthétiques pyrogénées peuvent également être utilisées. Les argiles, silicates et silices citées précédemment peuvent avantageusement être modifiées par des molécules organiques telles que des polyéthers, des amides éthoxylées, des sels d'ammonium quaternaires, des diamines à longue chaîne, des esters à longue chaîne, des polyéthylène glycols, des polypropylène glycols.

Ces particules peuvent être utilisées seules ou mélangées entre elles.

Selon un mode de réalisation, la composition C3 comprend au moins un polymère de poids moléculaire supérieur à 5 000 g.mol$^{-1}$ et des particules solides. Tout mélange des composés cités précédemment peut être utilisé.

### Etape b)

Dans l'étape b), l'émulsion (E2) ou l'émulsion (E'2), constituée de gouttes polydisperses dispersées dans une phase continue, est soumise à un cisaillement, par exemple dans un mélangeur, à une vitesse de cisaillement comprise entre 100 s$^{-1}$ et 100 000 s$^{-1}$.

Selon un mode de réalisation, la vitesse de cisaillement appliquée à l'étape b) est comprise entre 100 s$^{-1}$ et 50 000 s$^{-1}$.

De préférence, la vitesse de cisaillement appliquée à l'étape b) est comprise entre 1 000 s$^{-1}$ et 20 000 s$^{-1}$.

Pendant l'étape b), l'émulsion (E2) ou (E'2) est introduite dans un mélangeur et est ensuite soumise à un cisaillement qui résulte en la formation d'une troisième émulsion, l'émulsion (E3) ou (E'3), respectivement.

Comme la viscosité de la composition C3 est inférieure à 10 000 mPa.s à la vitesse de cisaillement à laquelle est réalisée l'étape b), l'émulsion (E2) ou (E'2) peut aisément être mélangée avec tout type d'agitateur. Ainsi pour réaliser l'étape b), on peut utiliser tout type d'agitateur usuellement utilisé pour former des émulsions, comme par exemple un agitateur mécanique à pâles, un émulseur statique, un homogénéisateur à ultrasons, un homogénéisateur à membrane, un homogénéisateur à haute pression, un moulin colloïdal, un disperseur à haut pouvoir de cisaillement ou un homogénéisateur à haute vitesse.

L'émulsion (E3) ou (E'3) est chimiquement identique à l'émulsion (E2) (ou (E'2) respectivement) mais elle est constituée de gouttes monodisperses, et non polydisperses comme (E2) ou (E'2).

Typiquement, l'émulsion (E'3) consiste en une dispersion de gouttes doubles comprenant un cœur constitué d'une ou plusieurs gouttes simples de composition C1, et une couche de composition C2 entourant ledit cœur, lesdites gouttes doubles étant dispersées dans la composition C3.

La différence entre l'émulsion (E2) (ou (E'2)) et l'émulsion (E3) (ou (E'3)) est la variation de taille des gouttes : les gouttes de l'émulsion (E2) (ou (E'2)) sont polydisperses en taille alors que les gouttes de l'émulsion (E3) (ou (E'3)) sont monodisperses grâce au mécanisme de fragmentation ayant lieu durant l'étape c).

Les gouttes d'émulsion (E2) (ou (E'2)) ne peuvent être fragmentées efficacement en des gouttes fines et monodisperses d'émulsion (E3) (ou (E'3)) que si une contrainte de cisaillement élevée leur est appliquée.

La contrainte de cisaillement σ appliquée à une goutte d'émulsion (E2) (ou (E'2)) est définie comme la force tangentielle par unité de surface de goutte résultant du cisaillement macroscopique appliqué à l'émulsion lors de son agitation au cours de l'étape c).

La contrainte de cisaillement σ (exprimée en Pa), la viscosité de la composition C3 η (exprimée en Pa.s) et la vitesse de cisaillement γ (exprimée en s$^{-1}$) appliquée à l'émulsion (E2) (ou (E'2)) lors de son agitation au cours de l'étape c) sont reliées par l'équation suivante :

$$\sigma = \eta\gamma$$

Ainsi, la combinaison de la viscosité de la composition C3 et du cisaillement appliqué par le mélangeur permet d'appliquer une haute contrainte de cisaillement aux gouttes d'émulsion (E2) (ou (E'2)) et ainsi de les fragmenter en des gouttes monodisperses.

*Etape c)*

L'étape c) consiste à soumettre l'émulsion (E3) (ou (E'3)) à une polymérisation, ce qui va permettre la polymérisation de la composition C2.

Selon un mode de réalisation de l'invention, pendant l'étape c), les gouttes simples d'émulsion (E3) constituées de composition C2 réticulable, sont réticulées et ainsi converties en des microparticules polymériques viscoélastiques.

Selon un autre mode de réalisation, pendant l'étape c), l'enveloppe des gouttes doubles d'émulsion (E'3), constituée de composition C2 réticulable, est réticulée et ainsi convertie en une enveloppe polymérique viscoélastique, encapsulant et protégeant l'actif de sa libération en l'absence d'une sollicitation mécanique.

Pendant l'étape c), l'émulsion (E3) (ou (E'3)) est laissée au repos pendant une période pouvant aller jusqu'à 100 heures. Pendant cette durée, de nombreux phénomènes de déstabilisation d'émulsion bien connus de l'homme de l'art peuvent rendre l'émulsion (E3) (ou (E'3)) à nouveau polydisperse, comme la coalescence, le mûrissement, la sédimentation ou le crémage. Une caractéristique importante de la présente invention est de supprimer ces phénomènes pendant toute la durée de l'étape c). En effet, comme la viscosité de la composition C3 est supérieure à 10 000 mPa.s à une vitesse de cisaillement de 10 s$^{-1}$, la mobilité des gouttes d'émulsion (E3) ou (E'3) est très fortement réduite et la cinétique de déstabilisation de l'émulsion est très lente par rapport à la durée de l'étape c).

Ainsi, la viscosité très élevée de la composition C3 à une vitesse de cisaillement de 10 s$^{-1}$ assure la stabilité de l'émulsion (E3) ou (E'3) jusqu'à ce que l'étape c) soit achevée.

Selon l'invention, le terme « polymérisation » englobe toute polymérisation ne nécessitant pas d'être initiée par exposition à une source de lumière. En particulier, la polymérisation selon l'invention n'est pas photoinitiée.

La polymérisation selon l'invention peut par exemple être initiée par exposition à la chaleur (initiation thermique), ou par la simple mise en contact des réactifs entre eux, ou par la simple mise en contact des réactifs avec un catalyseur.

Selon un mode de réalisation, le procédé de l'invention ne comprend pas d'étape d'exposition à une source de lumière UV.

Selon un mode de réalisation, l'étape c) de polymérisation n'est pas une étape de photopolymérisation.

Selon un mode de réalisation, l'étape c) de polymérisation de la composition C2 est effectuée pendant une durée comprise entre 8 heures et 100 heures.

Selon un mode de réalisation, l'étape c) de polymérisation de la composition C2 est effectuée à une température comprise entre 20°C et 80°C.

De préférence, l'étape c) de polymérisation de la composition C2 est effectuée pendant une durée comprise entre 8 heures et 100 heures, à une température comprise entre 20°C et 80°C.

La composition obtenue à l'issue de l'étape c), comprenant des microparticules ou des microcapsules solides dispersées dans la composition C3, est prête à l'emploi et peut être utilisée sans être lavée ou sans traitement supplémentaire.

L'épaisseur de l'enveloppe des microcapsules ainsi obtenues est typiquement comprise entre 10 nm et 2,5 μm, de préférence entre 100 nm et 1 000 nm.

Selon un mode de réalisation, les microparticules ou les microcapsules solides obtenues à l'issue de l'étape c) sont dépourvues d'eau et/ou de tensioactif.

Le procédé de l'invention présente l'avantage de ne pas nécessiter d'eau, dans aucune des étapes décrites. Le procédé de l'invention permet ainsi d'encapsuler des composés sensibles à l'eau.

Le procédé de l'invention présente l'avantage de ne pas nécessiter de tensioactif, dans aucune des étapes décrites. Le procédé de l'invention permet ainsi de réduire la présence d'additifs qui pourraient modifier les propriétés du produit final obtenu après libération de l'actif.

## EXEMPLES

### Exemple 1 : Préparation de microparticules solides

Cet exemple démontre : (i) l'utilisation d'une composition C3 gélifiée permettant de fabriquer des gouttes de taille inférieure à 20 μm puis d'empêcher la déstabilisation de l'émulsion pendant 48 heures ; (ii) l'obtention de microparticules réticulées après 48 heures.

*Composition de C'2 et C3 :*

- La composition C'2 est une préparation de PDMS apte à être polymérisée préparée avec le kit bicomposants Sylgard 184 fabriqué par Dow Corning. Le composant n°1 du kit comprend les produits suivants (données communiquées par Dow Corning) :

  ○ entre 55,0% et 75,0% de dimethyl, methylhydrogen siloxane,
  ○ entre 15,0% et 35,0% de dimethyl siloxane, dimethylvinyl-terminated,
  ○ entre 10,0% et 30,0% de dimethylvinylated and trimethylated silica,
  ○ entre 1,0% et 5,0% de tetramethyl tetravinyl cyclotetrasiloxane,
  ○ moins de 0,10% d'éthylbenzène.

Le composant n°2 du kit comprend les produits suivants (données communiquées par Dow Corning) :

  ○ moins de 200 ppm d'un complexe de platine,
  ○ entre 55,0 et 75,0% dimethyl siloxane, dimethylvinyl-terminated,
  ○ entre 30,0 et 50,0% dimethylvinylated and trimethylated silica,
  ○ moins de 1,0% tetra(trimethylsiloxy) silane,
  ○ 0,5% xylène,
  ○ 0,2% éthylbenzène.

Ce kit peut être considéré comme le mélange de plusieurs polymères, de plusieurs réticulants et d'un catalyseur au sens de l'invention.
La composition C'2 comprend 15% du composant n°1 et 85% du composant n°2.

- La composition C3 est une solution de carbomère Tego 340FD (Evonik, Essen, Germany) à 4% en masse dans de l'eau distillée. La solution est agitée à 2 000 rpm pendant 20 minutes à l'aide d'un agitateur à hélice. Le pH est ensuite ajusté à 6 à l'aide d'une solution d'hydroxyde de sodium 2M. La viscosité de la composition C3 à 10 s$^{-1}$ est 52 000 mPa.s.

*Fabrication des microparticules :*

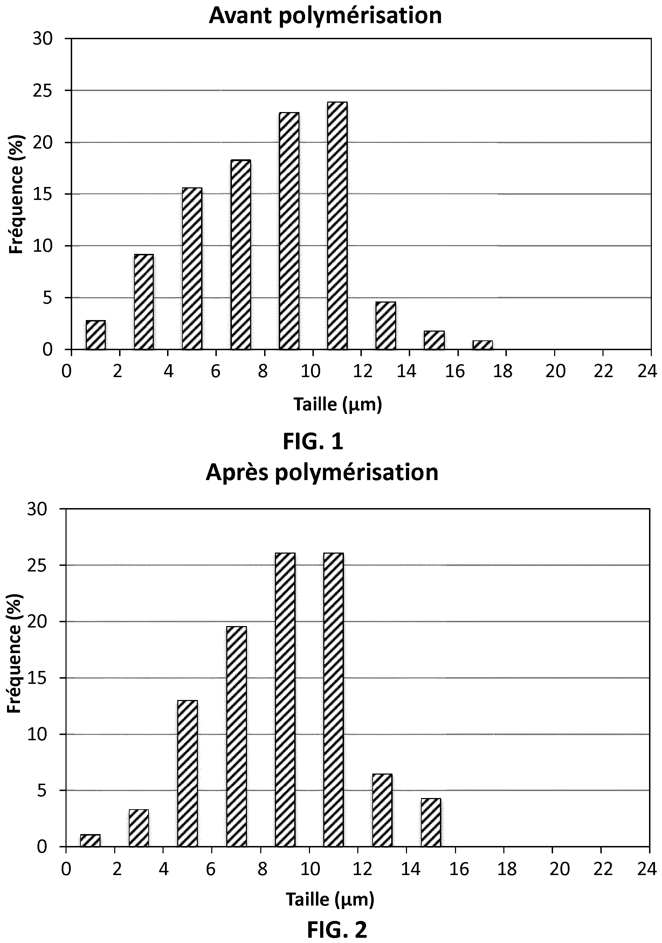

Etape a") : La composition C'2 est ajoutée goutte à goutte à la composition C3 sous agitation à 500 rpm jusqu'à obtenir un ratio C'2:C3 = 10:90 en poids. L'agitateur utilisé pour cette étape est un agitateur mécanique (Heidolph RZR 2021) équipé d'une hélice de type défloculeuse de diamètre 3 cm.
Etape b) : L'émulsion (E2) obtenue à l'étape précédente est introduite dans un mélangeur de type Couette fabriquée par l'entreprise TSR33. Ce mélangeur est composé de 2 cylindres concentriques, l'un mobile et l'autre fixe, séparés par un entrefer de 100 μm. La rotation du cylindre mobile permet d'appliquer un cisaillement uniforme à toute l'émulsion contenue dans l'entrefer. L'émulsion (E2) est soumise à un cisaillement de 1 000 s$^{-1}$. A cette vitesse de cisaillement, la viscosité de C3 n'est plus que de 2 500 mPa.s, ce qui permet à l'émulsion (E2) de transiter facilement à l'intérieur du mélangeur.
Etape c) : L'émulsion E3) ainsi obtenue est laissée pendant 48 heures au repos pour permettre la polymérisation des particules.
Les particules sont imagées par microscopie optique à contraste d'interférence avant et après l'étape c). Une analyse d'image réalisée avec le logiciel Image J permet d'en obtenir la distribution de taille. Les Figures 1 et 2 représentent les distributions de taille des capsules obtenues à différentes étapes du procédé de l'invention.
Avant l'étape c), les gouttes d'émulsion (E3) ont une taille moyenne de 8 μm et la largeur de la distribution de taille présente une largeur à mi-hauteur (considérée comme un moyen simple d'évaluer la monodispersité) de 5 μm (Figure 1). Après l'étape c), les particules polymérisées ont une taille moyenne de 8 μm et la largeur de la distribution de taille présente une largeur à mi-hauteur de 5 μm (Figure 2).
La distribution de taille est donc la même avant et après les 48 heures de polymérisation : l'émulsion (E3) n'a subi aucune déstabilisation grâce à la viscosité élevée de la composition C3.

### Exemple 2 : Préparation de microcapsules solides

Cet exemple démontre : (i) l'utilisation d'une composition C3 gélifiée afin de laisser reposer les doubles gouttes obtenues à la fin de l'étape de cisaillement pendant 48 heures sans aucune déstabilisation ; (ii) l'obtention de capsules réticulées après 48 heures.

*Composition de C1, C2 et C3 :*

- La composition C1 est un mélange d'huiles de paraffine (Sigma, St. Louis, MS) avec Aerosil 816 (Essen, Allemagne) (actif) à 4% en masse.
- La composition C2 est une préparation de PDMS apte à être polymérisée préparée avec le kit bicomposants Sylgard 184 de Dow Corning. La composition C2 comprend 30% du composant n°1 et 70% du composant n°2.

- La composition C3 est une solution de carbomère Tego 340FD (Evonik, Essen, Germany) à 4% en masse dans de l'eau distillée. La solution est agitée à 2 000 rpm pendant 20 minutes à l'aide d'un agitateur à hélice. Le pH est ensuite ajusté à 6 à l'aide d'une solution d'hydroxyde de sodium 2M. La viscosité de la composition C3 à 10 s$^{-1}$ est 52 000 mPa.s.

*Fabrication des microcapsules :*

Un agitateur mécanique (Heidolph RZR 2021) équipé d'une hélice d'agitation de type défloculeuse de diamètre 3 cm est utilisé pour réaliser toutes les étapes d'émulsification.

Etape a1) : la composition C1 est ajoutée goutte à goutte à la composition C2 sous agitation à 200 rpm jusqu'à obtenir un ratio C1:C2 = 20:80 en poids. L'agitation est ensuite maintenue à 200 rpm pendant 30 minutes.

Etape a2) : l'émulsion (E1) ainsi obtenue est ajoutée goutte à goutte à la composition C3 sous agitation à 500 rpm jusqu'à obtenir un ratio E1:C3 = 10:90 en poids.

Etape b) : L'émulsion (E2) ainsi obtenue est agitée à 500 rpm pendant 20 minutes. Dans ces conditions, le cisaillement appliqué à l'émulsion (E2), bien que très peu contrôlé, peut être estimé à moins de 500 s$^{-1}$ (pour le détail du calcul, on se référera à : Metzner AB, Otto RE. Agitation of non-Newtonian fluids. AIChE J (1957) 3: 3-10 ; Wu, J et al., Estimation of agitator flow shear rate. AIChE J (2006) 52: 2323-2332).

Etape c) : l'émulsion (E3) ainsi obtenue est laissée pendant 48 heures au repos sans agitation pour permettre la polymérisation des capsules.

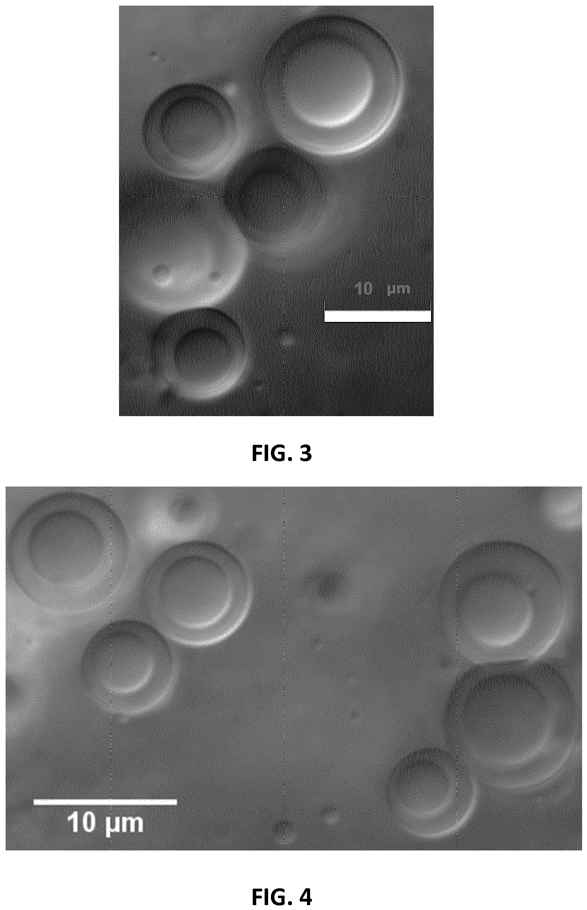

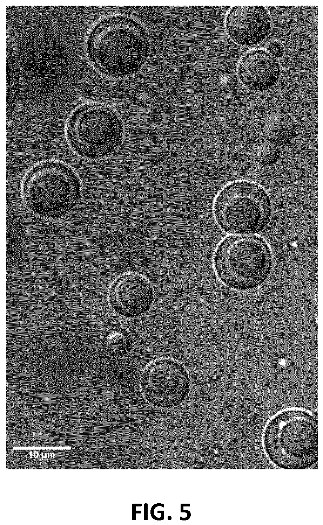

Les Figures 3 à 5 représentent des clichés de microscopie optique à contraste d'interférence prises à différentes étapes de la fabrication.

La Figure 3 représente des doubles gouttes d'émulsion (E3) après l'étape b).

La Figure 4 représente des capsules polymérisées après l'étape c).

La Figure 5 représente des capsules polymérisées imagées après avoir subi un cisaillement mécanique entre une lame et une lamelle de microscope, frottées l'une contre l'autre. Les capsules sont intactes après cette étape, ce qui montre qu'elles sont effectivement polymérisées.

Une analyse d'image réalisée avec le logiciel Image J permet d'obtenir la distribution de taille des capsules aux différentes étapes de la fabrication. Après l'étape b), les doubles gouttes ont une taille moyenne de 6,0 μm et la largeur de la distribution de taille présente une largeur à mi-hauteur (considérée comme un moyen simple d'évaluer la monodispersité) de 5,5 μm. Après l'étape c), les capsules polymérisées ont une taille moyenne de 5,8 μm et la largeur de la distribution de taille présente une largeur à mi-hauteur de 5,0 μm. La distribution de taille est donc la même avant et après les 48 heures de polymérisation : l'émulsion (E3) n'a subi aucune déstabilisation grâce à la viscosité élevée de la composition C3.

**Exemple 3** : **Préparation de microparticules solides selon l'invention**

Cet exemple démontre : (i) l'utilisation d'une composition C3 gélifiée au sens de l'invention permettant de fabriquer des gouttes de taille inférieure à 20 μm puis d'empêcher la déstabilisation de l'émulsion pendant 48 heures ; (ii) l'obtention de microparticules réticulées après 48 heures.

*Composition de C'2 et C3 :*

- La composition C'2 est une préparation de PDMS apte à être polymérisée préparée avec le kit bicomposants Sylgard 184 de Dow Corning. La composition C'2 comprend 15% du composant n°1 et 85% du composant n°2.
- La composition C3 est une solution d'alginate à 10% en masse. La viscosité de la composition C3 est 16 980 mPa.s à 10 s$^{-1}$ et 6 670 mPa.s à 100 s$^{-1}$. Cette composition C3 est donc gélifiée au sens de l'invention.

*Fabrication des microparticules :*

Etape a") : La composition C'2 est ajoutée goutte à goutte à la composition C3 sous agitation à 500 rpm jusqu'à obtenir un ratio C'2:C3 = 10:90 en poids. L'agitateur utilisé pour cette étape est un agitateur mécanique (Heidolph RZR 2021) équipé d'une hélice de type défloculeuse de diamètre 3 cm.

Etape b) : L'émulsion (E2) obtenue à l'étape précédente est agitée à 500 tpm pendant 10 minutes.

Etape c) : L'émulsion E3) ainsi obtenue est laissée pendant 48 heures au repos pour permettre la polymérisation des particules.

Les particules sont imagées par microscopie optique à contraste d'interférence avant et après l'étape c). Une analyse d'image réalisée avec le logiciel Image J permet d'en obtenir la distribution de taille.

Avant l'étape c), les gouttes d'émulsion (E3) ont une taille moyenne de 6,5 μm et la largeur de la distribution de taille présente une largeur à mi-hauteur de 3 μm. Après l'étape c), les particules polymérisées ont une taille moyenne de 7 μm et la largeur de la distribution de taille présente une largeur à mi-hauteur de 3 μm.

La distribution de taille est donc la même avant et après les 48 heures de polymérisation : l'émulsion (E3) n'a subi aucune déstabilisation grâce à la viscosité élevée de la composition C3.

**Exemple 4 (comparatif)** : Préparation de microparticules solides

Cet exemple démontre l'utilisation d'une composition C3 non gélifiée au sens de l'invention ne permettant pas d'empêcher la déstabilisation de l'émulsion pendant 48 heures.

*Composition de C'2 et C3 :*

- La composition C'2 est une préparation de PDMS apte à être polymérisée préparée avec le kit bicomposants Sylgard 184 de Dow Corning. La composition C'2 comprend 15% du composant n°1 et 85% du composant n°2.
- La composition C3 est une solution d'alginate à 5% en masse. La viscosité de la composition C3 est 1 340 mPa.s à 10 s$^{-1}$ et 890 mPa.s à 100 s$^{-1}$. Cette composition C3 n'est donc pas gélifiée au sens de l'invention.

*Fabrication des microparticules :*

Etape a") : La composition C'2 est ajoutée goutte à goutte à la composition C3 sous agitation à 500 tpm jusqu'à obtenir un ratio C'2:C3 = 10:90 en poids. L'agitateur utilisé pour cette étape est un agitateur mécanique (Heidolph RZR 2021) équipé d'une hélice de type défloculeuse de diamètre 3 cm.

Etape b) : L'émulsion (E2) obtenue à l'étape précédente est agitée à 500 tpm pendant 10 minutes.

Etape c) : L'émulsion E3) ainsi obtenue est laissée pendant 48 heures au repos pour permettre la polymérisation des particules.

Les particules sont imagées par microscopie optique à contraste d'interférence avant et après l'étape c). Une analyse d'image réalisée avec le logiciel Image J permet d'en obtenir la distribution de taille. Avant l'étape c), les gouttes d'émulsion (E3) ont une taille moyenne de 15 μm et la largeur de la distribution de taille présente une largeur à mi-hauteur de 11 μm. Après l'étape c), les particules polymérisées ont une taille moyenne de 31 μm et la largeur de la distribution de taille présente une largeur à mi-hauteur de 12 μm.

L'émulsion (E3) a donc subi une déstabilisation importante due aux propriétés non satisfaisantes de C3.

**Revendications**

1. Procédé de préparation de microparticules solides comprenant les étapes suivantes :

   a) l'addition sous agitation d'une composition C'2 dans une composition C3, les compositions C'2 et C3 n'étant pas miscibles l'une dans l'autre,

   la composition C'2 étant soit une composition monophasique réticulable soit une émulsion (E1) comprenant des gouttes d'une composition C1, comprenant au moins un actif, dispersées dans une composition polymérique C2 réticulable, les compositions C1 et C2 n'étant pas miscibles l'une dans l'autre,
   la viscosité de la composition C3 étant supérieure à 10 000 mPa.s à 25°C à une vitesse de cisaillement de 10 s$^{-1}$ et étant inférieure à 10 000 mPa.s à 25°C à une vitesse de cisaillement comprise entre 100 s$^{-1}$ et 100 000 s$^{-1}$,
   ce par quoi on obtient une émulsion (E2) comprenant des gouttes de composition C'2 dispersées dans la composition C3 ;

   b) l'application d'un cisaillement à l'émulsion (E2), ladite vitesse de cisaillement appliquée étant comprise entre 100 s$^{-1}$ et 100 000 s$^{-1}$,
   ce par quoi on obtient une émulsion (E3) comprenant des gouttes de composition C'2 de taille contrôlée dispersées dans la composition C3 ; et

   c) la polymérisation de la composition C'2, ce par quoi on obtient des microcapsules solides dispersées dans la composition C3, et pendant l'étape c), l'émulsion (E3) est laissée au repos pendant une période pouvant aller jusqu'à 100 heures.

2. Procédé selon la revendication 1, dans lequel la composition C'2 est une émulsion (E1) obtenue par l'addition sous agitation d'une composition C1, comprenant au moins un actif, dans une composition polymérique C2, les compositions C1 et C2 n'étant pas miscibles l'une dans l'autre.

3. Procédé selon la revendication 2, comprenant les étapes suivantes :

   a1) l'addition sous agitation d'une composition C1, comprenant au moins un actif, dans une composition polymérique C2, les compositions C1 et C2 n'étant pas miscibles l'une dans l'autre, ce par quoi on obtient une émulsion (E1) comprenant des gouttes de composition C1 dispersées dans la composition C2 ;

   a2) l'addition sous agitation de l'émulsion (E1) dans une composition C3, les compositions C2 et C3 n'étant pas miscibles l'une dans l'autre,

   la viscosité de la composition C3 étant supérieure à 10 000 mPa.s à 25°C à une vitesse de cisaillement de 10 s$^{-1}$ et étant inférieure à 10 000 mPa.s à 25°C à une vitesse de cisaillement comprise entre 100 s$^{-1}$ et 100 000 s$^{-1}$,

ce par quoi on obtient une émulsion double (E'2) comprenant des gouttes dispersées dans la composition C3 ;

b) l'application d'un cisaillement à l'émulsion (E'2), ladite vitesse de cisaillement appliquée étant comprise entre 100 s$^{-1}$ et 100 000 s$^{-1}$, ce par quoi on obtient une émulsion double (E'3) comprenant des gouttes de taille contrôlée dispersées dans la composition C3 ; et

c) la polymérisation de la composition C2, ce par quoi on obtient des microcapsules solides dispersées dans la composition C3, et pendant l'étape c), l'émulsion (E'3) est laissée au repos pendant une période pouvant aller jusqu'à 100 heures.

4. Procédé selon l'une quelconque des revendications 1 à 3, dans lequel la composition C2 est un liquide dont la viscosité à 25°C est comprise entre 500 mPa.s et 100 000 mPa.s.

5. Procédé selon l'une quelconque des revendications 1 à 4, dans lequel la composition C2 comprend au moins un monomère ou polymère, au moins un agent réticulant, et éventuellement au moins un catalyseur.

6. Procédé selon l'une quelconque des revendications 1 à 5, dans lequel la composition C2 ne comprend pas de photoinitiateur.

7. Procédé selon la revendication 5 ou 6, dans lequel la composition C2 comprend de 0,001% à 70% en poids d'agent réticulant par rapport au poids total de ladite composition.

8. Procédé selon l'une quelconque des revendications 1 à 7, dans lequel l'actif est solubilisé dans la composition C1 ou est dispersé sous la forme de particules solides dans la composition C1.

9. Procédé selon l'une quelconque des revendications 1 à 8, dans lequel la composition C3 comprend au moins un polymère branché, de préférence de poids moléculaire supérieur à 5 000 g.mol$^{-1}$, et/ou au moins un polymère de poids moléculaire supérieur à 5 000 g.mol$^{-1}$, et/ou des particules solides telles que des silicates.

10. Procédé selon l'une quelconque des revendications 1 à 9, dans lequel la viscosité de la composition C3 à 25°C est comprise entre 15 000 mPa.s et 100 000 mPa.s à une vitesse de cisaillement de 10 s$^{-1}$ et inférieure à 5 000 mPa s à une vitesse de cisaillement comprise entre 100 s$^{-1}$ et 100 000 s$^{-1}$.

11. Procédé selon l'une quelconque des revendications 1 à 10, dans lequel la vitesse de cisaillement appliquée à l'étape b) est comprise entre 10 s$^{-1}$ et 1 000 s$^{-1}$.

12. Procédé selon l'une quelconque des revendications 1 à 11, ne comprenant pas d'étape d'exposition à une source de lumière UV.

13. Procédé selon l'une quelconque des revendications 1 à 12, dans lequel l'étape de polymérisation de la composition C2 est effectuée pendant une durée comprise entre 8 heures et 100 heures.

14. Procédé selon l'une quelconque des revendications 1 à 13, dans lequel l'étape de polymérisation de la composition C2 est effectuée à une température comprise entre 20°C et 80°C.

15. Procédé selon l'une quelconque des revendications 1 à 14, dans lequel l'étape de polymérisation de la composition C2 est effectuée pendant une durée comprise entre 8 heures et 100 heures, à une température comprise entre 20°C et 80°C.

**Patentansprüche**

1. Verfahren zur Herstellung von festen Mikropartikeln, umfassend die folgenden Schritte:

a) Hinzufügen unter Rühren einer Zusammensetzung C'2 in eine Zusammensetzung C3, wobei die Zusammensetzungen C'2 und C3 nicht miteinander mischbar sind,

wobei die Zusammensetzung C'2 entweder eine vernetzbare einphasige Zusammensetzung oder eine Emulsion (E1) ist, umfassend Tropfen einer Zusammensetzung C1, umfassend mindestens einen Wirkstoff, die in einer vernetzbaren Polymerzusammensetzung C2 dispergiert sind, wobei die Zusammensetzungen C1 und C2 nicht miteinander mischbar sind, wobei die Viskosität der Zusammensetzung C3 bei 25 °C bei einer Schergeschwindigkeit von 10 s$^{-1}$ größer ist als 10.000 mPa.s und bei 25 °C bei einer Schergeschwindigkeit zwischen 100 s$^{-1}$ und 100.000 s$^{-1}$ kleiner ist als 10.000 mPa.s, wodurch eine Emulsion (E2) erlangt wird, umfassend Tropfen der Zusammensetzung C'2, dispergiert in der Zusammensetzung C3,

b) Anwenden einer Scherung auf die Emulsion (E2), wobei die angewendete Schergeschwindigkeit zwischen 100 s$^{-1}$ und 100.000 s$^{-1}$ ist, wodurch eine Doppelemulsion (E3) erlangt wird, umfassend Tropfen der Zusammensetzung C'2 kontrollierter Größe, dispergiert in der Zusammensetzung C3; und

c) Polymerisieren der Zusammensetzung C'2, wodurch feste Mikrokapseln dispergiert in der Zusammensetzung C3 erlangt werden, und wobei während Schritt c) die Emulsion (E3) über einen Zeitraum bis zu 100 Stunden ruhen gelassen wird.

2. Verfahren nach Anspruch 1, wobei die Zusammensetzung C'2 eine Emulsion (E1) ist, die durch Hinzufügen einer Zusammensetzung C1, umfassend mindestens einen Wirkstoff, in eine Polymerzusammensetzung C2 unter Rühren erlangt wird, wobei die Zusammensetzungen C1 und C2 nicht ineinander mischbar sind.

3. Verfahren nach Anspruch 2, umfassend die folgenden Schritte:

a1) Hinzufügen unter Rühren einer Zusammensetzung C1, umfassend mindestens einen Wirkstoff, in eine Polymerzusammensetzung C2 unter Rühren erlangt wird, wobei die Zusammensetzungen C1 und C2 nicht miteinander mischbar sind, wodurch eine Emulsion (E1) erlangt wird, umfassend Tropfen der Zusammensetzung C1, dispergiert in der Zusammensetzung C2;

a2) Hinzufügen unter Rühren der Emulsion (E1) in eine Zusammensetzung C3, wobei die Zusammensetzungen C2 und C3 nicht miteinander mischbar sind,

wobei die Viskosität der Zusammensetzung C3 bei 25 °C bei einer Schergeschwindigkeit von 10 s$^{-1}$ größer ist als 10.000 mPa.s und bei 25 °C bei einer Schergeschwindigkeit zwischen 100 s$^{-1}$ und 100.000 s$^{-1}$ kleiner ist als 10.000 mPa.s, wodurch eine Doppelemulsion (E'2) erlangt wird, umfassend Tropfen, die in der Zusammensetzung C3 dispergiert sind;

b) Anwenden einer Scherung auf die Emulsion (E'2), wobei die angewendete Schergeschwindigkeit zwischen 100 s$^{-1}$ und 100.000 s$^{-1}$ ist, wodurch eine Doppelemulsion (E'3) erlangt wird, umfassend Tropfen kontrollierter Größe, dispergiert in der Zusammensetzung C3; und

c) Polymerisieren der Zusammensetzung C2, wodurch feste Mikrokapseln dispergiert in der Zusammensetzung C3 erlangt werden, und wobei während Schritt c) die Emulsion (E'3) über einen Zeitraum bis zu 100 Stunden ruhen gelassen wird.

4. Verfahren nach einem der Ansprüche 1 bis 3, wobei die Zusammensetzung C2 eine Flüssigkeit ist, deren Viskosität bei 25 °C zwischen 500 mPa.s und 100.000 mPa.s ist.

5. Verfahren nach einem der Ansprüche 1 bis 4, wobei die Zusammensetzung C2 mindestens ein Monomer oder Polymer, mindestens ein Vernetzungsmittel und optional mindestens einen Katalysator umfasst.

6. Verfahren nach einem der Ansprüche 1 bis 5, wobei die Zusammensetzung C2 keinen Photoinitiator umfasst.

7. Verfahren nach Anspruch 5 oder 6, wobei die Zusammensetzung C2 0,001 Gewichts-% bis 70 Gewichts-% Vernetzungsmittel, bezogen auf das Gesamtgewicht der Zusammensetzung, umfasst.

8. Verfahren nach einem der Ansprüche 1 bis 7, wobei der Wirkstoff in der Zusammensetzung C1 solubilisiert oder in Form fester Teilchen in der Zusammensetzung C1 dispergiert ist.

9. Verfahren nach einem der Ansprüche 1 bis 8, wobei die Zusammensetzung C3 mindestens ein verzweigtes Polymer, vorzugsweise mit einem Molekulargewicht von mehr als 5.000 g.mol$^{-1}$, und/oder mindestens ein Polymer mit einem Molekulargewicht von mehr als 5.000 g.mol$^{-1}$, und/oder feste Partikel, wie beispielsweise Silikate, umfasst.

10. Verfahren nach einem der Ansprüche 1 bis 9, wobei die Viskosität der Zusammensetzung C3 bei 25 °C zwischen 15.000 mPa.s und 100.000 mPa.s bei einer Schergeschwindigkeit von 10 s$^{-1}$ ist und weniger als 5000 mPa s bei einer Schergeschwindigkeit zwischen 100 s$^{-1}$ und 100.000 s$^{-1}$ ist.

11. Verfahren nach einem der Ansprüche 1 bis 10, wobei die in Schritt b) angewandte Schergeschwindigkeit zwischen 10 s$^{-1}$ und 1.000 s$^{-1}$ ist.

12. Verfahren nach einem der Ansprüche 1 bis 11, das keinen Schritt eines Belichtens mit einer UV-Lichtquelle umfasst.

13. Verfahren nach einem der Ansprüche 1 bis 12, wobei der Schritt eines Polymerisierens der Zusammensetzung C2 über einen Zeitraum zwischen 8 Stunden und 100 Stunden durchgeführt wird.

**14.** Verfahren nach einem der Ansprüche 1 bis 13, wobei der Schritt eines Polymerisierens der Zusammensetzung C2 bei einer Temperatur zwischen 20 °C und 80 °C durchgeführt wird.

**15.** Verfahren nach einem der Ansprüche 1 bis 14, wobei der Schritt eines Polymerisierens der Zusammensetzung C2 über einen Zeitraum von 8 Stunden bis 100 Stunden bei einer Temperatur von 20 °C bis 80 °C durchgeführt wird.

**Claims**

**1.** Method for preparing solid microcapsules comprising the steps of:

a) the addition, with stirring, of a composition C'2 in a composition C3, the compositions C'2 and C3 not being miscible with each other, the composition C'2 being either a cross-linkable monophasic composition or an emulsion (E1) comprising drops of a composition C1, comprising at least one active ingredient, dispersed in a crosslinkable polymeric composition C2,

the compositions C1 and C2 not being miscible in each other, the viscosity of composition C3 being greater than 10,000 mPa.s at 25°C at a shear rate of 10 s$^{-1}$ and being less than 10,000 mPa.s at 25°C at a shear rate of between 100 s$^{-1}$ and 100,000 s$^{-1}$, wherein an emulsion (E2) comprising drops of composition C'2 dispersed in composition C3, is obtained;

b) applying a shear to the emulsion (E2), the applied shear rate being between 100 s$^{-1}$ and 100,000 s$^{-1}$, wherein an emulsion (E3) is obtained comprising controlled-size drops of composition C'2 dispersed in the composition C3; and
c) the polymerization of the composition C'2, wherein solid microcapsules dispersed in the composition C3 are obtained, and wherein during step c), the emulsion (E3) is allowed to stand for a period of up to 100 hours.

**2.** Method according to claim 1, in which the composition C'2 is an emulsion (E1) obtained by the addition, with stirring, of a composition C1 comprising at least one active ingredient, in a polymeric composition C2, the compositions C1 and C2 being immiscible in one another.

**3.** Method according to claim 2, comprising the steps of:

a1) the addition, with stirring, of a composition C1, comprising at least one active ingredient, in a polymeric composition C2, the compositions C1 and C2 not being miscible with each other, wherein an emulsion (E1) comprising drops of composition C1 dispersed in composition C2 is obtained;
a2) the addition, with stirring, of the emulsion (E1) in a composition C3, the compositions C2 and C3 not being miscible with each other, the viscosity of composition C3 being greater than 10,000 mPa.s at 25°C at a shear rate of 10 s$^{-1}$ and being less than 10,000 mPa.s at 25°C at a shear rate of between 100 s$^{-1}$ and 100,000 s$^{-1}$, wherein a double emulsion (E'2) is obtained comprising drops dispersed in the composition C3;
b) applying shear to the emulsion (E'2), the applied shear rate being between 100 s$^{-1}$ and 100,000 s$^{-1}$, wherein a double emulsion (E'3) is obtained comprising controlled-size drops dispersed in the composition C3; and
c) the polymerization of the composition C2, wherein solid microcapsules dispersed in the composition C3 are obtained, and wherein during step c), the emulsion (E'3) is allowed to stand for a period of up to 100 hours.

**4.** Method according to any one of claims 1 to 3, wherein the composition C2 is a liquid the viscosity of which at 25°C is between 500 mPa.s and 100 000 mPa.s.

**5.** Method according to any one of claims 1 to 4, wherein the composition C2 comprises at least one monomer or polymer, at least one crosslinking agent, and optionally at least one catalyst.

**6.** Method according to any one of claims 1 to 5, wherein the composition C2 does not include a photoinitiator.

**7.** Method according to claim 5 or 6, wherein the composition C2 comprises from 0.001% to 70% by weight of crosslinking agent relative to the total weight of the composition.

**8.** Method according to any one of claims 1 to 7, wherein the active ingredient is solubilized in the composition C1 or is dispersed in the form of solid particles in the composition C1.

**9.** Method according to any one of claims 1 to 8, wherein the composition C3 comprises at least one branched polymer, preferably with a molecular weight greater than 5,000 g.mol$^{-1}$, and/or at least one polymer of molecular weight greater than 5,000 $g.mol^{-1}$, and/or solid particles such as silicates.

10. Method according to any one of claims 1 to 9, wherein the viscosity of the composition C3 at 25°C is between 15,000 mPa.s and 100,000 mPa.s at a shear rate of 10 $s^{-1}$ and less than 5,000 mPa.s at a shear rate of between 100 $s^{-1}$ and 100,000 $s^{-1}$.

11. Method according to any one of claims 1 to 10, wherein the shear rate applied in step b) is between 10 $s^{-1}$ and 1,000 $s^{-1}$.

12. Method according to any one of claims 1 to 11, not including a step of exposure to a UV light source.

13. Method according to any one of claims 1 to 12, wherein the polymerization step of the composition C2 is carried out for a period between 8 hours and 100 hours.

14. Method according to any one of claims 1 to 13, wherein the step of polymerizing the composition C2 is carried out at a temperature between 20°C and 80°C.

15. Method according to any one of claims 1 to 14, wherein the polymerization step of the composition C2 is carried out for a period of between 8 hours and 100 hours, at a temperature between 20°C and 80°C.

Avant polymérisation

FIG. 1

Après polymérisation

FIG. 2

**FIG. 3**

**FIG. 4**

**FIG. 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6335315 B
- US 5877145 A

**Littérature non-brevet citée dans la description**

- **FARID et al.** A review on phase change energy storage: materials and applications. *Energy Conversion and Management*, 2004, vol. 45 (9-10), 1597-1615
- **LIU et al.** *Polymer Chemistry*, 2013, vol. 4, 3431-3443
- **METZNER AB ; OTTO RE**. Agitation of non-Newtonian fluids. *AIChE J*, 1957, vol. 3, 3-10
- **WU, J et al.** Estimation of agitator flow shear rate. *AIChE J*, 2006, vol. 52, 2323-2332